# EUROPÄISCHE PATENTANMELDUNG

(11) **EP 1 813 190 A1**
(43) Veröffentlichungstag der Anmeldung: **01.08.2007**
(21) Anmeldenummer: 07100113.5
(22) Anmeldetag: 04.01.2007
(51) Int. Cl.: A61B 5/12, H04R 25/00

(54) **Hörtestgerät**

(30) Priorität: 30.01.2006 DE 102006004362
(71) Anmelder: Siemens Audiologische Technik GmbH, 91058 Erlangen (DE)
(72) Erfinder: Geiger, Wolfgang, 91611, Lehrberg (DE); Rass, Uwe, 90480, Nürnberg (DE); Ryman, Robert, Bucks, HP7 9ET (GB)
(74) Vertreter: Berg, Peter

(57) **Zusammenfassung**

Die Ressourcen-Planung in einer Klinik oder bei einem Hörgeräte-Akustiker soll vereinfacht werden. Hierfür werden ein Hörtestgerät sowie ein Verfahren zum Betrieb eines Hörtestgerätes vorgeschlagen, bei denen nach einem einfachen Hörtest neben der Information, ob bei einem Benutzer die Versorgung mit einem Hörgerät erforderlich ist, auch die ggf. vorhandene Hörverlust-Klasse anzeigt wird, in die der Benutzer eingruppiert werden kann. Dadurch lässt sich der zeitliche, materielle und personelle Aufwand leichter abschätzen, der bei dem jeweiligen Benutzer zur Anpassung eines Hörgerätes aufzubringen ist.

## Beschreibung

Die Erfindung betrifft ein Hörtestgerät mit Mitteln zur Stimulation des Gehörs eines Benutzers und Mitteln zum Erfassen einer Reaktion des Benutzers auf die Stimulation. Ferner betrifft die Erfindung ein Verfahren zum Betrieb eines Hörtestgerätes sowie ein Hörgerät.

Vor der Versorgung eines Benutzers mit einem Hörgerät wird zunächst dessen Hörverlust mit einem geeigneten Hörtestgerät ermittelt. Hierzu wird das Gehör mit Testsignalen verschiedener Signalfrequenzen und unterschiedlichen Schalldruckpegeln stimuliert und die Reaktion des Benutzers auf das jeweilige Testsignal erfasst. Anhand des Hörtest-Ergebnisses wählt ein Hörgeräte-Akustiker ein geeignetes Hörgerät zum Ausgleich des individuellen Hörverlustes aus. Moderne Hörgeräte lassen sich in ihrem Signalübertragungsverhalten durch Programmierung an den individuellen Hörverlust des Benutzers anpassen.

Aus der EP 1 073 314 A1 ist ein Verfahren zur Anpassung eines Hörhilfegerätes bekannt, bei dem ein Benutzer mit unterschiedlichen Testsignalen stimuliert wird, Reaktionen des Benutzers auf die Testsignale erfasst werden und hörgerätespezifische Anpassparameter für das Hörhilfegerät generiert und auf das Hörhilfegerät übertragen werden. Auch aus der US 5,197,332 ist ein derartiges Verfahren sowie ein zugehöriges Test- und Anpassgerät bekannt.

Aus der DE 88 03 680 U1 ist ein Audiometer mit vollautomatischer Benutzerführung bekannt, bei dem ein Computer am Ende eines Hörtests ein Audiogramm und eine kurze Beschreibung des Testergebnisses, etwa in der Form: "Sie hören auf dem linken Ohr nicht einwandfrei", ausdruckt.

Zur Versorgung eines Hörgeräteträgers stehen Hörgeräte aus unterschiedlichen Hörgeräte-Kategorien zur Verfügung, die in Anbetracht des individuellen Hörverlustes mehr oder weniger gut für die Versorgung eines konkreten Benutzers geeignet sind. Unterschiedliche Hörgeräte-Kategorien sind zum Beispiel in dem Ohr tragbare Hörgeräte, hinter dem Ohr tragbare Hörgeräte für eine offene Versorgung oder hinter dem Ohr tragbare Hörgeräte mit einer individuell ausgebildeten Otoplastik (geschlossene Versorgung). Bislang ist es Aufgabe des Hörgeräte-Akustikers, eine für den Benutzer in Frage kommende Hörgeräte-Kategorie auszuwählen. Dabei unterscheidet sich der zeitliche Aufwand für die Anpassung von Hörgeräten aus unterschiedlichen Kategorien an einen Benutzer erheblich.

Ziel der Erfindung ist es, die Ressourcen-Planung in einer Klinik oder bei einem Hörgeräte-Akustiker zu vereinfachen.

Diese Aufgabe wird durch ein Hörtestgerät mit den in Patentanspruch 1 aufgeführten Merkmalen gelöst. Ferner wird die Aufgabe durch ein Verfahren mit den in Patentanspruch 13 aufgezeigten Verfahrensschritten gelöst.

Das Hörtestgerät gemäß der Erfindung umfasst Mittel zur Stimulation des Gehörs eines Benutzers. Dies kann ein einfacher Tongenerator sein, mit dem einem Benutzer Testsignale unterschiedlicher Frequenz und Lautstärke über Lautsprecher oder einen Kopfhörer dargeboten werden. Das Gehör des Benutzers kann aber auch mit anderen Mitteln, beispielsweise Körperschall, stimuliert werden.

Das Hörtestgerät gemäß der Erfindung umfasst ferner Mittel zum Erfassen wenigstens einer Reaktion des Benutzers auf die jeweilige Stimulation. Dies kann beispielsweise ein Tastschalter sein, der von dem Benutzer gedrückt wird, sobald er einen Ton wahrnimmt. Es können aber auch andere Reaktionen des Benutzers, beispielsweise unwillentliche Reaktionen des Körpers wie otoakustische Emissionen, erfasst werden. Die erfassten Reaktion werden dann automatisch ausgewertet, derart, dass als Ergebnis der Auswertung der individuelle Hörverlust des Benutzers einer bestimmten Hörverlust-Klasse aus einer Anzahl möglicher Hörverlust-Klassen zugeordnet wird.

Eine einfache Möglichkeit der Einteilung von Hörverlusten in unterschiedliche Hörverlust-Klassen besteht in der Charakterisierung des Hörverlustes in unterschiedlichen Graden (z.B. "schwach", "mäßig" oder "stark") in unterschiedlichen Frequenzbereichen (z.B. Tieftonbereich, mittlerer Frequenzbereich, Hochtonbereich). Eine bestimmte Hörverlust-Klasse könnte so bestimmt sein durch einen mäßigen Hörverlust im Tieftonbereich, einen schwachen Hörverlust im mittleren Frequenzbereich und einem starken Hörverlust im Hochtonbereich. Es sind aber auch andere Unterscheidungsmerkmale unterschiedlicher Hörverlust-Klassen möglich. Zum Beispiel die Klasse der Hörverluste, die nur mit einem hinter dem Ohr tragbaren Hörgerät mit einem individuellen Ohrpassstück in geeigneter Weise versorgt werden kann, oder die Hörverlust-Klasse, die dadurch charakterisiert ist, dass auch eine offene Versorgung zum Ausgleich des Hörverlustes geeignet ist.

Das Hörtestgerät gemäß der Erfindung umfasst vorteilhaft eine optische Anzeige, durch die dem Benutzer das Ergebnis eines Hörtests angezeigt wird. Beispielsweise bedeutet das Aufleuchten eines roten Lichts, dass eine geschlossene Versorgung mit einem individuell angepassten Ohrpassstück erforderlich ist. Leuchtet hingegen ein orangefarbenes Licht, so bedeutet dies, dass auch eine offene Versorgung geeignet ist. Ein grünes Licht kann beispielsweise signalisieren, dass kein nennenswerter Hörverlust vorliegt und kein Hörgerät erforderliche ist. Natürlich besteht neben der genannten Anzeige eine Vielzahl weiterer Möglichkeiten, um das Ergebnis des Hörtests optisch (z.B. über ein Display), akustisch (z.B. mittels Sprachausgabe) oder taktil anzuzeigen.

Die Erfindung bietet den Vorteil, dass dem Benutzer durch einen schnell und einfach auszuführenden Hörtest nicht nur angezeigt wird, ob eine Versorgung mit einem Hörgerät empfehlenswert ist oder nicht, sondern auch die Hörverlust-Klasse angezeigt wird, in die der individuelle Hörverlust des Benutzers eingeordnet werden kann, so dass sogleich die Art der Versorgung (Hörgeräte-Kategorie) feststeht mit der der Benutzer in geeigneter Weise versorgt werden kann. Daraus kann wiederum der zeitliche, der personelle und auch der KostenAufwand abgeschätzt werden, der für die Anpassung eines Hörgerätes an diesen Benutzer erforderlich ist. Dies vereinfacht den Workflow bei der Hörgerät-Anpassung erheblich.

Die Erfindung ist insbesondere dann von Vorteil, wenn bei einem Hörscreening, also einer Reihenuntersuchung, eine große Anzahl von Personen innerhalb kurzer Zeit untersucht werden soll. Die Gruppe an Personen, für die keine Hörgeräte-Versorgung erforderlich ist, kann so schnell von der Gruppe von Personen mit verringertem Hörvermögen unterschieden werden. Bei der Gruppe der Personen mit verringertem Hörvermögen steht dann sogleich die für die jeweilige Person in Frage kommende Hörgeräte-Kategorie fest, so dass der für die Betreuung und Versorgung der jeweiligen Person mit einem Hörgerät erforderliche zeitliche, materielle und personelle Aufwand leichter kalkuliert werden kann.

Eine Weiterbildung der Erfindung sieht vor, dass von dem Hörtestgerät erzeugte Daten auf ein Hörgeräte-Anpassgerät oder direkt auf ein Hörgerät übertragbar sind. Das Hörtestgerät weist hierfür eine geeignete Schnittstelle auf. Vorzugsweise erfolgt die Datenübertragung jedoch drahtlos, so dass an dem Hörtestgerät entsprechende Sende- und/oder Empfangsmittel zur drahtlosen Signalübertragung vorhanden sind.

Es können sowohl die Einzel-Ergebnisse der jeweils durchgeführten Hörtests als auch Daten zur Kennzeichnung der ermittelten Hörverlust-Klasse oder auch daraus resultierende Daten, beispielsweise Hörgeräte-Parameter zur Anpassung der Signalverarbeitung im Hörgerät an den ermittelten Hörverlust, übertragen werden.

Bei der direkten Datenübertragung zu einem Hörgerät kann das betreffende Hörgerät somit auch ohne Anpassgerät und ohne Anpasssoftware eingestellt werden, was für Gebiete mit verminderter Infrastruktur nützlich ist.

Bei der Übertragung der Daten an ein Anpassgerät können eine geeignete Anpassstrategie und/oder geeignete Anpassparameter voreingestellt werden. Nachfolgend können komplexere Einstellungen und Optimierungen durch eine fachlich versierte Person vorgenommen werden.

Die Erfindung wird nachfolgend anhand eines Ausführungsbeispiels näher erläutert. Dabei zeigen:
Figur 1 ein Hörtestgerät gemäß der Erfindung und
Figur 2 das Ablaufschema eines mit dem Hörtestgerät durchgeführten Hörtests.

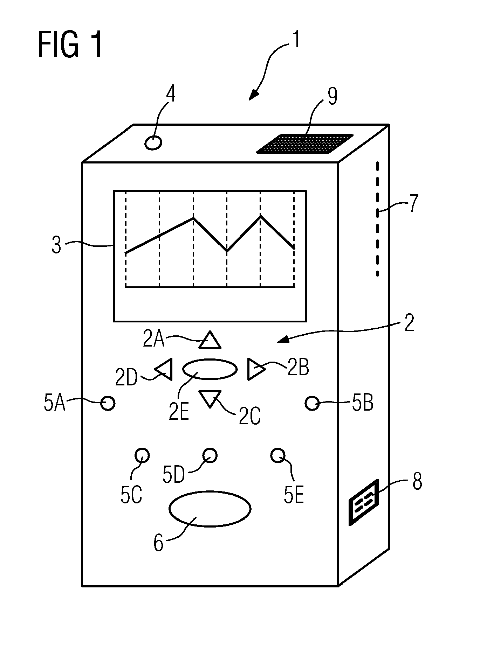

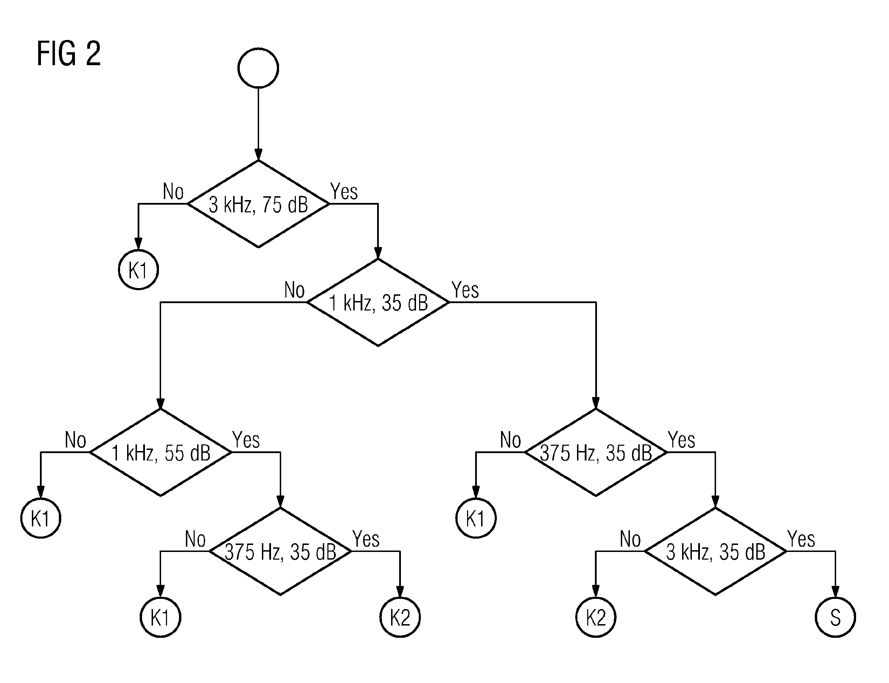

Das in Figur 1 gezeigte Hörtestgerät 1 umfasst eine Bedieneinrichtung 2 mit den Pfeiltasten 2A, 2B, 2C und 2D sowie einem zentriert zwischen den Pfeiltasten angeordneten Bedienelement 2E. Mit der Bedieneinrichtung 2 kann in Verbindung mit einem Display 3 unter anderem ein einfacher Hörtest ausgewählt und gestartet werden. Bei diesem Hörtest werden dem Benutzer Töne unterschiedlicher Frequenz und Lautstärke über einen Kopfhörer (nicht gezeichnet) zugeführt. Der Kopfhörer wird dabei an einer Anschlussbuchse 4 an dem Hörtestgerät 1 angeschlossen. Eine erste LED 5A leuchtet, sobald das Hörtestgerät 1 betriebsbereit ist. Eine zweite LED 5B signalisiert, dass gerade ein Test-Ton abgegeben wird. Der Test-Ton wird nur für eine kurze Zeitdauer, z.B. 3 Sekunden, abgegeben. Kann der Benutzer den abgegebenen Test-Ton hören, so bestätigt er dies sogleich durch Betätigung des Tastschalters 6. Kann er den Test-Ton nicht wahrnehmen, so erfolgt keine Betätigung des Tastschalters 6 oder eines anderen Bedienelements. Nach Ablauf einer kurzen Pause nach Abgabe des ersten Test-Tons wird ein zweiter Test-Ton mit gegenüber dem ersten Test-Ton veränderter Frequenz und/oder veränderter Lautstärke abgegeben. Das Ablaufschema einer möglichen Abfolge von Test-Tönen ist in Figur 2 veranschaulicht, auf die an späterer Stelle näher eingegangen wird. Es wird darauf hingewiesen, dass im Rahmen der Erfindung eine Vielzahl unterschiedlicher Abfolgen von Tönen, Geräuschen oder allgemein Testsignalen möglich ist, die jedoch so aufeinander abgestimmt sind, dass aus den Reaktionen des Benutzers letztendlich auf eine bestimmte Hörverlust-Klasse und damit auf die Möglichkeit, den individuellen Hörverlust des Benutzers mit einem Hörgerät einer bestimmten Hörgeräte-Kategorie in geeigneter Weise versorgen zu können, geschlossen werden kann. Bei dem Hörtestgerät 1 gemäß dem Ausführungsbeispiel sind eine rote LED 5C, eine orangefarbene LED 5D und eine grüne LED 5E vorhanden. Leuchtet bei Beendigung des Hörtests die rote LED 5C, so bedeutet dies, dass nur eine Versorgung mit einem Hörgerät mit einer individuell angepassten Otoplastik möglich ist. Leuchtet die orangefarbene LED 5D, so wird damit signalisiert, dass auch eine offene Versorgung möglich ist. Leuchtet die grüne LED 5E, so wird damit angezeigt, dass kein Hörgerät erforderlich ist.

Durch die Erfindung kann schnell und einfach abgeschätzt werden, welchen Typ Hörgerät ein bestimmter Benutzer benötigt, womit sich ungefähr auch der zur Anpassung eines entsprechenden Hörgerätes erforderliche zeitliche Aufwand abschätzen lässt.

Besonders vorteilhaft wirkt sich die Erfindung dann aus, wenn eine Vielzahl von Personen innerhalb kurzer Zeit untersucht und ggf. mit einem Hörgerät versorgt werden soll. Bei einem derartigen Hörscreening war bislang der zeitliche Aufwand für die Hörgeräte-Anpassung von einem Hörverlust betroffener Personen nur sehr schwer abzuschätzen. Bei der bevorzugten Ausführungsform eines Hörtestgerätes gemäß der Erfindung besteht die Möglichkeit der Datenübertragung mit weiteren Geräten, z.B. einem PC (nicht dargestellt). Bei dem Hörtestgerät 1 ist dies durch eine Antenne 7 sowie eine Buchse 8 angedeutet, durch die eine drahtlose Verbindung bzw. eine Kabelverbindung zu anderen Geräten ermöglicht wird. So lassen sich die bei den einzelnen Personen gewonnenen Daten an einen PC übermitteln und dort weiter verarbeiten. Dadurch können die erforderlichen Ressourcen besser geplant werden. Es ist sogar möglich, die Daten in ein elektronisch gesteuertes Workflow-Management-System einfließen zu lassen, wie es beispielsweise in einigen Kliniken unterhalten wird. Die Ressourcen-Planung kann dann weitgehend automatisiert erfolgen.

Es ist auch möglich, die mit dem Hörtestgerät 1 erzeugten Daten an ein Hörgeräte-Anpassgerät oder direkt an ein Hörgerät zu übertragen. Die Daten lassen sich dabei zum Beispiel für die erste Anpassung eines Hörgerätes (first fit) verwenden. Ferner ist es möglich, dass direkt in dem Hörtestgerät anhand der erzeugten Daten Hörgeräte-Anpassparameter generiert und an ein betreffendes Hörgerät übertragen werden zur Anpassung der Signalverarbeitung in dem Hörgerät an den individuellen Hörverlust eines Benutzers. Ein weiteres Hörgeräte-Anpassgerät ist dann nicht mehr erforderlich.

Das Hörtestgerät 1 gemäß dem Ausführungsbeispiel ist vorteilhaft mit einem Display 3 ausgestattet, mit dem beispielsweise der Hörverlust eines Benutzers in Abhängigkeit der Frequenz grafisch veranschaulicht werden kann. Darüber hinaus kann anhand des Displays 3 auch das Ergebnis eines Hörtests gemäß der Erfindung veranschaulicht werden. Dies ist insbesondere dann vorteilhaft, wenn eine sehr feine Einteilung möglicher Hörverluste in eine Vielzahl von Hörgeräte-Klassen vorgenommen wird. Ebenso kann auch eine sehr viel feinere Einteilung von Hörgeräten in unterschiedliche Kategorien vorgenommen werden. Allein bei den in dem Ohr Geräten kann beispielsweise unterschieden werden zwischen CIC-Geräten (completely in the canal), ITC-Geräten (in the canal) und Concha-Geräten, jeweils mit universell passender Gehäuseschale (universal fit) oder individuellem Gehäuse (custom fit). Eine Anzeige mittels LEDs wie in dem Ausführungsbeispiel wird dann unübersichtlich. Ferner ist es häufig möglich, dass ein bestimmter Hörverlust mit Hörgeräten unterschiedlicher Kategorien in geeigneter Weise versorgt werden kann. Auch dieser Sachverhalt lässt sich eventuell anhand einer Anzeige auf einem Display besser veranschaulichen.

Das Hörtestgerät 1 gemäß dem Ausführungsbeispiel weist weiterhin einen Lautsprecher 9 auf, über den ebenfalls Testsignale ausgegeben werden können oder das Ergebnis eines Hörtests akustisch angezeigt werden kann.

Für die äußere Gestaltung eines Hörtestgerätes gemäß der Erfindung steht eine Reihe an Alternativen zur Verfügung. So kann es sich bei dem Hörtestgerät, wie in Figur 1 veranschaulicht, um ein handhaltbares Gerät handeln. Es kommt aber auch ein größerer Aufbau, z.B. in Form eines Tischgerätes, in Betracht. Auch ein mehrteiliger Aufbau, z.B. mit einer separaten Anzeigeeinrichtung (Bildschirm) ist möglich.

Vorteilhaft ist das Hörtestgerät gemäß der Erfindung jedoch mit Geräten mit einer anderen Funktion baulich vereint bzw. in diese Geräte integriert. So kann das Hörtestgerät beispielsweise auch als Fernbedienung für ein Hörgerät, ja sogar als Hörgerät selbst ausgebildet sein. Auch die Integration der Funktionalität des Hörhilfegerätes gemäß der Erfindung in ein handelsübliches Audiometer ist denkbar. Weiterhin kann das Hörtestgerät gemäß der Erfindung auch in ein komplexes Computersystem, z.B. ein Workflow-Management-System für eine Klinik, integriert sein.

Figur 2 zeigt ein Ablaufschema eines beispielhaften Hörtests gemäß der Erfindung. Der Ablauf erfolgt in folgenden Schritten:
- Stimulation des Benutzers mit einem ersten akustischen Signal mit einer ersten Signalfrequenz und einem ersten Schalldruckpegel,
- Anzeige einer ersten Hörgeräte-Kategorie K1 durch das Hörtestgerät für den Fall, dass das erste akustische Signal von dem Benutzer nicht gehört wurde und Beendigung des Verfahrens,
- Stimulation des Benutzers mit einem zweiten akustischen Signal mit einer zweiten Signalfrequenz und einem zweiten Schalldruckpegel für den Fall, dass das erste Signal von dem Benutzer gehört wurde,
- Stimulation des Benutzers mit einem dritten akustischen Signal mit einer zweiten Signalfrequenz und einem dritten Schalldruckpegel für den Fall, dass das zweite akustische Signal von dem Benutzer nicht gehört wurde,
- Anzeige der ersten Hörgeräte-Kategorie K1 durch das Hörtestgerät für den Fall, dass das dritte akustische Signal von dem Benutzer nicht gehört wurde und Beendigung des Verfahrens,
- Stimulation des Benutzers mit einem vierten akustischen Signal mit einer dritten Signalfrequenz und einem zweiten Schalldruckpegel für den Fall, dass das dritte akustische Signal von dem Benutzer gehört wurde,
- Anzeige der ersten Hörgeräte-Kategorie K1 durch das Hörtestgerät für den Fall, dass das vierte akustische Signal von dem Benutzer nicht gehört wurde und Beendigung des Verfahrens,
- Anzeige einer zweiten Hörgeräte-Kategorie K2 durch das Hörtestgerät für den Fall, dass das vierte akustische Signal von dem Benutzer gehört wurde und Beendigung des Verfahrens,
- Stimulation des Benutzers mit einem fünften akustischen Signal mit einer dritten Signalfrequenz und einem zweiten Schalldruckpegel für den Fall, dass das zweite akustische Signal von dem Benutzer gehört wurde,
- Anzeige einer ersten Hörgeräte-Kategorie K1 durch das Hörtestgerät für den Fall, dass das fünfte akustische Signal von dem Benutzer nicht gehört wurde und Beendigung des Verfahrens,
- Stimulation des Benutzers mit einem sechsten akustischen Signal mit einer ersten Signalfrequenz und einem zweiten Schalldruckpegel für den Fall, dass das fünfte akustische Signal von dem Benutzer gehört wurde,
- Anzeige einer zweiten Hörgeräte-Kategorie K2 durch das Hörtestgerät für den Fall, dass das sechste akustische Signal von dem Benutzer nicht gehört wurde und Beendigung des Verfahrens,
- Anzeige eines Symbols S durch das Hörtestgerät für den Fall, dass das sechste akustische Signal von dem Benutzer gehört wurde und Beendigung des Verfahrens. Das Symbol S zeigt an, dass kein behandlungsbedürftiger Hörverlust vorliegt.

Bei dem Hörtestgerät entsprechend dem Ausführungsbeispiel gemäß Figur 1 wird durch eine rote LED signalisiert, dass ein schwerer Hörverlust vorliegt und die Versorgung mit einem Hörgerät einer ersten Kategorie, beispielsweise einem HdO-Hörgerät mit einer individuell angepassten Otoplastik, erforderlich ist. Eine orangefarbene LED signalisiert, dass ein leichter bis mittlerer Hörverlust vorliegt, der auch mit einer offenen Versorgung in geeigneter Weise behandelbar ist.

Für die erste Signalfrequenz kann z.B. 3kHz, für die zweite Signalfrequenz 1 kHz und für die dritte Signalfrequenz 375 Hz gewählt werden. Weiterhin kann für den ersten Schalldruckpegel z.B. 75dB, für den zweiten Schalldruckpegel 35dB und für den dritten Schalldruckpegel 55dB gewählt werden.

Es wird nochmals darauf hingewiesen, dass sowohl die angegebenen Zahlenwerte als auch die Auswahl und Abfolge der Testsignale und Testschritte an sich erheblich von dem Ausführungsbeispiel differieren können, ohne dadurch den Schutzbereich der Erfindung zu verlassen.

## Patentansprüche

1. Hörtestgerät mit
- Mitteln zur Stimulation des Gehörs eines Benutzers,
- Mitteln zum Erfassen einer Reaktion des Benutzers auf die Stimulation,
- Mitteln zur automatischen Auswertung der erfassten Reaktion derart, dass als Ergebnis der Auswertung der individuelle Hörverlust des Benutzers einer bestimmten, den Grad der Schwerhörigkeit kennzeichnenden Hörverlust-Klasse aus einer Anzahl möglicher Hörverlust-Klassen zugeordnet wird.

2. Hörtestgerät nach Anspruch 1 mit Mitteln zum Anzeigen der zugeordneten Hörverlust-Klasse.

3. Hörtestgerät nach Anspruch 1 oder 2 mit Mitteln zum Anzeigen wenigstens einer bestimmten Hörgeräte-Kategorie aus einer Anzahl möglicher Hörgeräte-Kategorien, wobei durch ein Hörgerät der angezeigten Kategorie eine geeignete Versorgung des Benutzers zum Ausgleich seines individuellen Hörverlustes möglich ist.

4. Hörtestgerät nach Anspruch 2 oder 3 mit einer optischen Anzeige zum Anzeigen der Hörverlust-Klasse bzw. der Hörgeräte-Kategorie.

5. Hörtestgerät nach Anspruch 3 oder 4, wobei die zur Versorgung des individuellen Hörverlustes am besten geeignete Hörgeräte-Kategorie angezeigt wird.

6. Hörtestgerät nach einem der Ansprüche 1 bis 5, wobei zur Stimulation des Gehörs akustische Signale mit verschiedenen Signalfrequenzen abgebbar sind.

7. Hörtestgerät nach einem der Ansprüche 1 bis 6, wobei zur Stimulation des Gehörs akustische Signale mit verschiedenen Schalldruckpegeln abgebbar sind.

8. Hörtestgerät nach einem der Ansprüche 1 bis 7 mit Mitteln zur Übertragung von Daten, die die ermittelte Hörverlust-Klasse kennzeichnen, an ein Hörgerät oder ein Hörgeräte-Anpassgerät.

9. Hörtestgerät nach einem der Ansprüche 1 bis 8 mit Mitteln zur Generierung von Hörgeräte-Einstellparametern aus den bezüglich des Hörverlustes ermittelten Daten und mit Mitteln zur Übertragung der Hörgeräte-Einstellparameter an ein Hörgerät.

10. Hörtestgerät nach einem der Ansprüche 8 oder 9, wobei die Ermittlung der Hörgeräte-Einstellparameter in Abhängigkeit der dem Benutzer zugeordneten Hörverlust-Klasse erfolgt.

11. Hörtestgerät nach einem der Ansprüche 1 bis 10, das zugleich als Fernbedienung für ein Hörgerät ausgebildet ist.

12. Hörgerät mit einem integrierten Hörtestgerät nach einem der Ansprüche 1 bis 10.

13. Verfahren zum Betrieb eines Hörtestgerätes nach einem der Ansprüche 1 bis 11 mit folgenden Schritten:
- Stimulation des Benutzers mit akustischen Signalen mit unterschiedlichen Signalfrequenzen und/oder mit unterschiedlichen Schalldruckpegeln,
- Erfassen der Reaktionen des Benutzers auf die unterschiedlichen akustischen Signale,
- Auswerten der Reaktionen des Benutzers auf die unterschiedlichen akustischen Signale,
- Ermitteln einer den Grad der Schwerhörigkeit kennzeichnenden Hörverlust-Klasse, in die der individuelle Hörverlust des Benutzers fällt,
- Anzeigen eines Testergebnisses in Abhängigkeit der ermittelten Hörverlust-Klasse.

14. Verfahren zum Betrieb eines Hörtestgerätes nach Anspruch 13, wobei eine Hörgeräte-Kategorie ermittelt und angezeigt wird, bei der sich ein Hörgerät aus dieser Kategorie zum Ausgleich des individuellen Hörverlustes des Benutzers eignet.

15. Verfahren zum Betrieb eines Hörtestgerätes nach Anspruch 13 oder 14 mit folgenden Schritten:
- Stimulation des Benutzers mit einem ersten akustischen Signal mit einer ersten Signalfrequenz und einem ersten Schalldruckpegel,
- Anzeige einer ersten Hörgeräte-Kategorie (K1) durch das Hörtestgerät für den Fall, dass das erste akustische Signal von dem Benutzer nicht gehört wurde und Beendigung des Verfahrens,
- Stimulation des Benutzers mit einem zweiten akustischen Signal mit einer zweiten Signalfrequenz und einem zweiten Schalldruckpegel für den Fall, dass das erste Signal von dem Benutzer gehört wurde,
- Stimulation des Benutzers mit einem dritten akustischen Signal mit einer zweiten Signalfrequenz und einem dritten Schalldruckpegel für den Fall, dass das zweite akustische Signal von dem Benutzer nicht gehört wurde,
- Anzeige der ersten Hörgeräte-Kategorie (K1) durch das Hörtestgerät für den Fall, dass das dritte akustische Signal von dem Benutzer nicht gehört wurde und Beendigung des Verfahrens,
- Stimulation des Benutzers mit einem vierten akustischen Signal mit einer dritten Signalfrequenz und einem zweiten Schalldruckpegel für den Fall, dass das dritte akustische Signal von dem Benutzer gehört wurde,
- Anzeige der ersten Hörgeräte-Kategorie (K1) durch das Hörtestgerät für den Fall, dass das vierte akustische Signal von dem Benutzer nicht gehört wurde und Beendigung des Verfahrens,
- Anzeige einer zweiten Hörgeräte-Kategorie (K2) durch das Hörtestgerät für den Fall, dass das vierte akustische Signal von dem Benutzer gehört wurde und Beendigung des Verfahrens,
- Stimulation des Benutzers mit einem fünften akustischen Signal mit einer dritten Signalfrequenz und einem zweiten Schalldruckpegel für den Fall, dass das zweite akustische Signal von dem Benutzer gehört wurde,
- Anzeige einer ersten Hörgeräte-Kategorie (K1) durch das Hörtestgerät für den Fall, dass das fünfte akustische Signal von dem Benutzer nicht gehört wurde und Beendigung des Verfahrens,
- Stimulation des Benutzers mit einem sechsten akustischen Signal mit einer ersten Signalfrequenz und einem zweiten Schalldruckpegel für den Fall, dass das fünfte akustische Signal von dem Benutzer gehört wurde,
- Anzeige einer zweiten Hörgeräte-Kategorie (K2) durch das Hörtestgerät für den Fall, dass das sechste akustische Signal von dem Benutzer nicht gehört wurde und Beendigung des Verfahrens,
- Anzeige eines Symbols (S) durch das Hörtestgerät für den Fall, dass das sechste akustische Signal von dem Benutzer gehört wurde und Beendigung des Verfahrens.

16. Verfahren nach Anspruch 15, wobei die erste Signalfrequenz in etwa 3kHz und die zweite Signalfrequenz in etwa 1 kHz und die dritte Signalfrequenz in etwa 375 Hz beträgt.

17. Verfahren nach Anspruch 15 oder 16, wobei der erste Schalldruckpegel in etwa 75dB und der zweite Schalldruckpegel in etwa 35dB und der dritte Schalldruckpegel in etwa 55dB beträgt.

18. Integration eines Hörtestgerätes nach einem der Ansprüche 1 bis 11 oder eines Verfahrens nach einem der Ansprüche 13 bis 17 in ein computergestütztes Workflow-Management-System.
